(19) Europäisches Patentamt
European Patent Office
Office européen des brevets

(11) Veröffentlichungsnummer: **0 627 214 A1**

# (12) EUROPÄISCHE PATENTANMELDUNG

(21) Anmeldenummer: **94107899.0**

(51) Int. Cl.$^{5}$: **A61K 7/42**

(22) Anmeldetag: **21.05.94**

(30) Priorität: **04.06.93 DE 4318576**

(43) Veröffentlichungstag der Anmeldung:
**07.12.94 Patentblatt 94/49**

(84) Benannte Vertragsstaaten:
**DE ES FR GB IT**

(71) Anmelder: **MERCK PATENT GmbH**
**Frankfurter Strasse 250**
**D-64293 Darmstadt (DE)**

(72) Erfinder: **Kurz, Thekla, Dr.**
**Schillerstrasse 47**
**D-64846 Gross-Zimmern (DE)**
Erfinder: **Stössel, Sieglinde**
**Königsbergerstrasse 8**
**D-64354 Reinheim (DE)**
Erfinder: **Spiller, Andrea**
**Bunsenstrasse 48/3**
**D-32657 Lemgo (DE)**

## (54) Hautfärbende Zubereitung.

(57) Die Erfindung betrifft eine hautfärbende Zubereitung, enthaltend eine Hydroxycarbonyl-Verbindung mit selbstbräunenden Eigenschaften, in einem kosmetisch verträglichen Träger, welche mindestens einen hauthaftenden Farbstoff enthält.

EP 0 627 214 A1

Die Erfindung betrifft eine hautfärbende Zubereitung, enthaltend eine Hydroxycarbonyl-Verbindung mit selbstbräunenden Eigenschaften, in einem kosmetisch verträglichen Träger, welche mindestens einen hauthaftenden Farbstoff enthält.

Es ist seit langem bekannt, daß Verbindungen, welche eine Ketol-Gruppierung

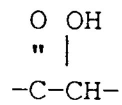

aufweisen, vorzugsweise Hydroxymethylketone, insbesondere Dihydroxyaceton aber auch Methylglyoxal, eine selbstbräunende Wirkung auf die menschliche Haut ausüben. Dieser selbstbräunende Effekt beruht im wesentlichen auf einer Maillard-Reaktion zwischen der Ketol-Gruppierung dieser Verbindungen und den Aminosäuren der Haut.

Desweiteren ist bekannt, daß die Farbtönung dieser Reaktion dabei durch Zusatz bestimmter Adjuvantien noch verstärkt werden kann.

So wird z.B. in der EP 04 56 545 und EP 05 00 446 vorgeschlagen, dem natürlichen Melamin nachgebildete Indol-Derivate in solchen Formulierungen einzusetzen. Diese müsen jedoch aufgrund ihrer geringen Hauthaftung in relativ hohen Konzentrationen eingesetzt werden.

Nach der FR 20 85 208 können mit Formulierungen enthaltend Dihydroxyaceton und einen wasserlöslichen Farbstoff künstliche Sommersprossen erzeugt werden. Aufgrund der Wasserlöslichkeit dieser Farbstoffe ist die Hauthaftung jedoch nur gering.

Desweiteren wurde vorgeschlagen, Formulierungen zur Selbstbräunung (z.B. U.S.P. 3, 920, 808 oder U.S.P. 4, 708, 865) herzustellen, die neben Dihydroxyaceton ungesättigte Ketone z.B. Lawson oder Juglon enthalten. Diese erzeugen jedoch einen unnatürlichen wirkenden, gelblichen Farbton.

Es wurde nun festgestellt, daß sich ein der natürlichen Bräunung ähnliche Hautfärbung von länger Beständigkeit erzielen läßt, wenn die hautfärbende Zubereitung neben einer Hydroxycarbonylverbindung mindestens einen hauthaftenden Farbstoff enthält.

Aufgabe der vorliegenden Erfindung war es nun, hautfärbende Zubereitungen bereitzustellen, welche einen, für einen großen Zeitraum bestehenden, der natürlichen Bräunung ähnlichen Farbton hervorrufen. Es wurde nun überraschenderweise gefunden, daß solche hautfärbenden Zubereitungen erhalten werden, wenn man ihnen hauthaftende Farbstoffe zusetzt.

Bevorzugte Ausführungsformen sind:

a) Hautfärbende Zubereitungen, wobei die Verbindung mit selbstbräunenden Eigenschaften Dihydroxyaceton oder Methylglyoxal ist.

b) Hautfärbende Zubereitungen, wobei der hauthaftende Farbstoff ein roter Farbstoff ist.

c) Hautfärbende Zubereitungen, wobei der hauthaftende Farbstoff ein Eosin-Derivat ist, vorzugsweise der Formel I ist,

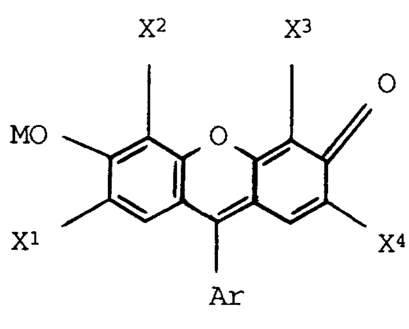

(I)

worin

$X^1$ bis $X^4$ jeweils unabhängig voneinander H, $NO_2$ oder Halogen, vorzugsweise Br oder J, M, H oder ein Alkalimetall, vorzugsweise Natrium, und Ar eine durch eine Metallcarboxylatgruppe, substituierte Phenylgruppe, die gegebenenfalls durch 1 bis 4 Halogenatome substituiert sein kann, bedeuten.

d) Hautfärbende Zubereitungen, welche 0,1 bis 10, vorzugsweise 1 bis 6 Gew.-% einer hautfärbenden Verbindung, und 0,01 bis 0,1, vorzugsweise etwa 0,05 Gew.-% mindestens eines hauthaftenden Farbstoffes jeweils bezogen auf die gesamte Zubereitung enthalten.

e) Hautfärbende Zubereitungen, wobei das Gewichtsverhältnis zwischen dem hauthaftenden Farbstoff und der hautfärbenden Verbindung größer als 0,01 ist.

f) Hautfärbende Zubereitunge, wobei der kosmetisch verträgliche Träger ausgewählt ist aus der Gruppe bestehend aus:
Wasser;
einem Gemisch aus Wasser und einem oder mehreren organischen Komponenten;
einem Gemisch aus Fetten und einem oder mehreren organischen Lösungsmitteln.

g) Hautfärbende Zubereitungen, wobei die organischen Komponenten oder Lösungsmittel ausgewählt sind aus der Gruppe bestehend aus:
den kurzkettigen $C_{1-4}$-Alkoholen, den langkettigen $C_{10-18}$-Monoalkholen, den $C_{2-8}$-Polyalkoholen, den Alkylenglykolen, den Glykolethern, den Alkylacetaten, den $C_{1-3}$-Monoalkylethern des Ethylenglykols und den Estern der gesättigten $C_{14-16}$-Fettsäuren, insbesondere Ethanol, Cetylalkohol, Propylenglykol, Ethylenglykolmonoethylether und Ethylenglykolmonobutylether.

h) Hautfärbende Zubereitungen, wobei die Fette ausgewählt sind aus der Gruppe bestehend aus: pflanzlichen und tierischen Ölen, Mineralölen, Fettsäuren, Fettalkoholen, Fettsäureestern und Fettsäuretriglyceriden.

i) Hautfärbende Zubereitungen, wobei der pH-Wert zwischen 2 und 10, vorzugsweise zwischen 3 und 6, liegt:

j) Hautfärbende Zubereitungen, welche mindestens einen UV-Filter zum Schutz vor UV-A und/oder UV-B Strahlen enthalten.

k) Hautfärbende Zubereitungen, welche oberflächenaktive Mittel, feuchtigkeitsspendende Mittel, Linderungsmittel, Trübungsmittel, Dihydroxyindol-Derivate, Vitamine, Parfüme, Gelbildner, Verdickungsmittel, Pigmente oder Konservierungsmittel enthalten.

Die Erfindung betrifft weiterhin ein Verfahren zur Färbung der Haut mit einem der natürlichen Bräunung ähnlichem Farbton, dadurch gekennzeichnet, daß man eine Zubereitung gemäß einem der Ansprüche 1 bis 9 auf die Haut aufbringt, insbesondere wobei die Zubereitung mit Pigmenten mit anderen Farbstoffen, welche den Farbton des hauthaftenden Farbstoffes abdecken, vorzugsweise Tartrazin (C.I. 19140), Solagenbrillant schwarz (C.I. 28440), Brillantblau Acid Blue q (C.I. 42090) oder Solvent violett (C.I. 60725) auf einen, vorzugsweise braunen Make-up Farbton abgemischt ist.

In einer besonders bevorzugten Ausführungsform wird der Farbton des hauthaftenden Farbstoffes durch natürliche, gefärbte Kosmetikwirkstoffe abgedeckt. Zu diesen zählen insbesondere blaue Azulene, wie Guajazulen oder Chamuzulen und gelbe Flavonoide, wie Rutin oder Quercetin.

Bevorzugte Verbindungen mit selbstbräunenden Eigenschaften sind solche mit einer Ketol-Gruppierung;

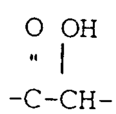

insbesondere Dihydroxyaceton, Methylglyoxal, Glycerinaldehyd, Erythrulose, Alloxan, 2,3-Dihydroxysuccindialdehyd, 2,3-Dimethoxysuccindialdehyd,2-Amino-3-hydroxysuccindialdehyd oder 2-Benzylamino-3-hydroxysuccindialdehyd.

Bevorzugte hauthaftende Farbstoffe sind Eosin-Derivate der Formel I, insbesondere Eosin, Erythrosin, Rose bengale, Phloxin, Cyanosin, Daphinin, Eosin G, Eosin 10B, Acid Red 51, insbesondere Eosin G oder Erythrosin.

Wird die erfindungsgemäße kosmetische Zubereitung als Mittel zur Bräunung menschlicher Epidermis verwendet, so liegt es in verschiedenen, für diesen Typ üblicherweise verwendeten Formen vor. So kann es insbesondere in Form öbliger oder ölig-alkoholischer Lotionen, Emulsione, wie als Creme oder als Milch in Form ölig-alkoholischer, ölig-wäßriger oder wäßrig-alkoholischer Gele oder als feste Stifte vorliegen oder als Aerosol konditioniert sein.

Es kann kosmetische Adjuvanzien enthalten, welche in dieser Art von Mittel üblicherweise verwendet werden, wie z.B. Verdickungsmittel, weichmachende Mittel, Befeuchtungsmittel, grenzflächenaktive Mittel, Konservierungsmittel, Mittel gegen Schaumbildung, Parfums, Wachse, Lanolin, Treibmittel, Farbstoffe

und/oder Pigmente, welche das Mittel selbst färben, UV-Filter zum Schutz vor UV-A und/oder UV-B-Strahlen und andere in der Kosmetik gewöhnlich verwendete Ingredienzien.

Man kann als Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugte Ausführungsform der Erfindung ist eine Emulsion, welche als Creme oder -milch vorliegt und außer der Verbindung der Formel I Fettalkohole, Fettsäureester, insbesondere Triglyceride von Fettsäuren, Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen Ölen und Wachsen, Lanolin; Fettsäureestern, insbesondere Triglyceriden von Fettsäuren, oder ölig-alkoholische Lotionen auf Basis eines Niedrigalkohols, wie Ethanol, oder eines Glycols, wie Propylenglykol und/oder eines Polyols, wie Glycerin, und Ölen, Wachsen und Fettsäureestern, wie Triglyceriden von Fettsäuren, dar.

Das erfindungsgemäße kosmetische Mittel kann auch als alkoholisches Gel vorliegen, welches einen oder mehrere Niedrigalkohole oder -polyole, wie Ethanol, Propylenglycol oder Glycerin, und ein Verdikkungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäureestern, Lanolin und anderen Fettkörpern.

Gegenstand der Erfindung sind auch kosmetische Sonnenschutzmittel, die mindestens eine $\alpha$-Hydroxycarbonyl-Verbindung mit selbstbräunenden Eigenschaften, mindestens einen hauthaftenden Farbstoff und UVB- und/oder UVA-Filter umfassen können.

Geeignete UV-Filter sind zum Beispiel Derivate der Zimtsäure, Benzylidencampher und seine Derivate, p-Aminobenzoesäure und seine Derivate, Salizylsäurederivate, Benzophenonderivate und Dibenzoylmethanderivate. Diese UV-Filter sind, in der Regel, zu 0,2 bis 10 Gew.-%, bezogen auf die Verbindungen mit selbstbräunenden Eigenschaften, in den Zubereitungen enthalten.

Ist ein Mittel als Aerosol konditioniert, verwendet man in der Regel die üblichen Treibmittel, wie Alkane, Fluoralkane und Chlorfluoralkane.

Die erfindungsgemäße Zubereitung kann gegebenenfalls Verdickungsmittel enthalten:
Zum Verdicken können die dem Fachmann geläufigen Verdickungsmittel oder Gelbildner verwendet werden, wie z.B. Guargummi, Heterobiopolysaccharide, Xanthangummi, Skleroglucane, Derivate der Cellulose, wie z.B. Methylcellulose, Hydroxyethylcellulose, Hydroxymethylpropylcellulose, Alkalisalze der Carboxymethylcellulose und Polyacrylsäuren.

Da der hauthaftende Farbstoff bereits in der Formulierung und auf der Haut eine Eigenfarbe aufweist, die nicht erst bei der Entwicklung der Selbstbräunung entsteht, ist es besonders vorteilhaft, durch Zugabe von Pigmenten oder anderen Farbstoffen, die mit dem hauthaftenden Farbstoff einen braunen Make-up Ton ergeben, den Farbton des hauthaftenden Farbstoffes zu verdecken.

Ein weiterer Gegenstand der Erfindung sind kosmetische Zubereitungen, welche

a) eine $\alpha$-Hydroxycarbonyl-Verbindung mit selbstbräunenden Eigenschaften, vorzugsweise 0,1 bis 10 Gew.%, bezogen auf die Zubereitung,

b) einen hauthaftenden Farbstoff, vorzugsweise 0,01 bis 0,1 Gew.%, bezogen auf die Zusammensetzung,

c) ein Farbpigment, insbesondere ein Effekt-Pigment basierend auf einem Metalloxid belegten plättchenförmigen Substrat, vorzugsweise 1 bis 15 Gew.%, bezogen auf die Zusammensetzung, oder

d) einen anderen Farbstoff, insbesondere einen Farbstoff, welcher den Farbton des hauthaftenden Farbstoffes abdeckt, vorzugsweise 0,01 bis 0,1 Gew.%, bezogen auf die Zusammensetzung,

e) gegebenenfalls Titandioxid, vorzugsweise 1 bis 10 Gew.%, bezogen auf die Zusammensetzung, enthalten.

Auch ohne weitere Ausführung wird davon ausgegangen, daß ein Fachmann die obige Beschreibung in weitestem Umfang nutzen kann. Die nachfolgenden Beispiele sind deswegen lediglich als beschreibende, keineswegs als in irgendeiner Weise limitierende Offenbarung aufzufassen.

Die vollständige Offenbarung aller vor- und nachstehenden Anmeldungen, Patente und Veröffentlichungen, ist durch Bezugnahme in diese Anmeldung eingeführt.

Beispiel 1: Getönte Selbstbräunungsmilch

| | | | % |
|---|---|---|---|
| A | Arlatone 983 S<br>Arlatone 985<br>Brij 76<br>Paraffinöl flüssig (Art.-Nr. 7162)<br>Miglyol 812 | (2)<br>(2)<br>(2)<br>(1)<br>(3) | 1,50<br>2,50<br>1,50<br>5,00<br>5,00 |
| B | Karion F flüssig (Art.-Nr. 2993)<br>Propandiol-1,2 (Art.-Nr. 7478)<br>Eosin G 1%ige Lsg. (Art.-Nr. 15935)<br>Konservierungsmittel<br>Wasser, demineralisiert | (1)<br>(1)<br>(1)<br>(1) | 2,50<br>2,50<br>5,00<br>q.s.<br>ad 100,00 |
| C | Dihydroxyaceton (Art.-Nr. 10150)<br>Wasser, demineralisiert | (1)<br>(1) | 5,00<br>5,00 |
| D | Pigment Transparent Yellow Ochre (Art.-Nr. 17381) | (1) | 7,00 |
| | Pigment Transparent Black (Art.-Nr. 17161) | (1) | 1,00 |
| | Titandioxid 1171 | (4) | 6,00 |

Herstellung:

Phase A auf 75 °C, Phase B auf 80 °C erhitzen. Phase B langsam in Phase A einrühren. Homogenisieren. Unter Rühren abkühlen, bei 40 °C Phase C zugeben. Zum Schluß langsam die Pigemente unterrühren.

Bemerkungen:

Muster enthalten als Konservierungsmittel
0,05 % Propyl-4-hydroxybenzoat (Merck-Art.-Nr. 7427)
0,15 % Methyl-4-hydroxybenzoat (Merck-Art.-Nr. 6757)

Bezugsquellen:

1. E. Merck, Darmstadt
2. ICI, Essen
3. Hüls Troisdorf AG, Witten
4. Kronos Titan GmbH, Leverkusen

Anwendungsbeispiel 1:

Die nach Beispiel 1 hergestellte Zusammensetzung wird bei verschiedenen Probanden aufgetragen.

Die so erhaltenen Farbtöne werden nach 21,5 Stunden (Tabelle 1) und 45 Stunden (Tabelle 2) nach der sogenannten Lab-Methode (z.B. nach Ullmann's Encyclopedia of Industriel Chemistry, Vol A 20, S. 262-263, 1992, Verlag Chemie) ausgewertet.

L = Helligkeit
a = Rotanteil
b = Gelbanteil
C = Farbintensität
h = Farbton (Bunttonwinkel)

und mit dem entsprechenden Farbtönen ohne hautfärbende Zubereitung bzw. mit hautfärbenden Zubereitung ohne hauthaftenden Farbstoff verglichen.

Die erfindungsgemäße Zubereitung erzielt einen wesentlich höheren Rotanteil auch nach längerer Einwirkungszeit und mehrmaligem Waschen bei etwa gleicher Farbintensität (C).

Tabelle 1

**Werte 21,5 h nach dem Auftragen**

| Haut | leer | | | | |
|---|---|---|---|---|---|
| Proband | L | a | b | C | h |
| 1 | 61,616 | 7,453 | 13,715 | 15,6091 | 61,4771 |
| 2 | 60,909 | 6,336 | 12,769 | 14,254 | 63,6102 |
| 3 | 60,891 | 5,65 | 12,39 | 13,6177 | 65,4868 |
| 4 | 52,605 | 8,12 | 17,264 | 19,0783 | 64,8117 |
| 5 | 70,802 | 4,384 | 18,105 | 18,6284 | 76,3887 |
| 6 | | | | | |
| 7 | 65,032 | 4,844 | 11,888 | 12,8369 | 67,8308 |
| 8 | 63,583 | 6,633 | 14,003 | 15,4949 | 64,6539 |
| *9 | 58,032 | 9,233 | 17,99 | 20,221 | 62,8332 |
| 10 | 63,251 | 5,678 | 13,373 | 14,5286 | 66,9963 |
| 11 | 66,761 | 7,523 | 15,351 | 17,0952 | 63,8931 |
| 12 | 68,987 | 3,288 | 8,341 | 8,9656 | 68,486 |

**Nach Auftragen einer Zubereitung mit 5,4 % DHA**

| Proband | L | a | b | C | h |
|---|---|---|---|---|---|
| 1 | 57,91 | 9,8 | 19,017 | 21,3935 | 62,7351 |
| 2 | 55,621 | 9,475 | 17,817 | 20,1795 | 61,9943 |
| 3 | 59,368 | 8,913 | 16,726 | 18,9526 | 61,9493 |
| 4 | 53,898 | 11,147 | 19,593 | 22,5425 | 60,3635 |
| 5 | 62,783 | 9,07 | 20,896 | 22,7792 | 66,5364 |
| 6 | 60,458 | 8,744 | 16,567 | 18,7327 | 62,1761 |
| 7 | 65,635 | 6,313 | 14,599 | 15,9051 | 66,615 |
| 8 | 60,517 | 9,366 | 17,503 | 19,8514 | 61,85 |
| 9 | 52,019 | 11,077 | 23,282 | 25,7824 | 64,5564 |
| 10 | 58,205 | 9,643 | 18,881 | 21,201 | 62,9458 |
| 11 | 52,914 | 11,443 | 20,881 | 23,8111 | 61,2762 |
| 12 | 63,747 | 8,059 | 17,46 | 19,2298 | 65,2241 |

**Nach Auftragen einer Zubereitung mit 4,0 % DHA + 0,05 % Eosin**

| Proband | L | a | b | C | h |
|---|---|---|---|---|---|
| 1 | 60,415 | 10,547 | 16,661 | 19,56 | 58,4074 |
| 2 | 65,729 | 10,578 | 16,019 | 19,1966 | 56,5625 |
| 3 | 61,63 | 9,389 | 15,792 | 18,3724 | 59,2686 |
| 4 | 53,327 | 12,512 | 19,143 | 22,8694 | 56,8318 |
| 5 | 60,481 | 7,399 | 16,752 | 18,3135 | 66,1703 |
| 6 | 58,744 | 8,195 | 14,804 | 16,9205 | 61,0328 |
| 7 | 64,915 | 7,607 | 14,611 | 16,4726 | 62,4972 |
| 8 | 56,53 | 9,517 | 17,313 | 19,757 | 61,2019 |
| 9 | 52,775 | 10,482 | 21,949 | 24,3238 | 64,473 |
| 10 | 57,883 | 10,267 | 15,987 | 18,9998 | 57,2921 |
| 11 | 57,932 | 13,085 | 17,936 | 22,2018 | 53,8894 |
| 12 | 65,478 | 6,853 | 13,667 | 15,2813 | 63,3696 |

**Vergleichswert; Proband 13 nach Sommerurlaub**

| | L | a | b | C | h |
|---|---|---|---|---|---|
| Arm dunkel | 49,5908 | 10,662 | 18,294 | 21,1745 | 59,7654 |
| Arm hell | 53,241 | 10,089 | 18,339 | 20,9308 | 61,184 |

* Proband 9 war erst seit 2 Wochen aus dem Urlaub zurück

Tabelle 2

**Werte 45 h nach dem Auftragen**

| DHA | | | DHA+Eosin | | |
|---|---|---|---|---|---|
| Proband | C | h | Proband | C | h |
| 1 | 22,1871 | 64,1365 | 1 | 20,8885 | 62,8327 |
| 2 | 18,4367 | 65,4451 | 2 | 18,8088 | 61,9299 |
| 3 | 18,5933 | 64,3664 | 3 | 19,831 | 62,4987 |
| 4 | 23,6082 | 60,5514 | 4 | 21,5169 | 59,5625 |
| 5 | 22,7578 | 67,9486 | 5 | 21,1785 | 67,964 |
| 6 | 18,6948 | 62,0513 | 6 | 15,9307 | 63,283 |
| 7 | 15,876 | 72,3164 | 7 | 15,694 | 68,1098 |
| 8 | 18,4847 | 61,6671 | 8 | 19,0126 | 60,972 |
| 9 | 23,6388 | 64,5476 | 9 | 22,6928 | 63,9565 |
| 10 | 20,6111 | 63,216 | 10 | 17,7356 | 57,3972 |
| 11 | 19,0048 | 62,8249 | 11 | 19,6395 | 62,4783 |
| 12 | 18,04 | 66,0599 | 12 | 14,8434 | 68,4706 |

Anwendungsbeispiel 2:

Eine nach Beispiel 1 hergestellte Zusammensetzung wird bei einem Probanden aufgetragen und jeweils mit einer Zusammensetzung verglichen, die anstelle Eosin G Lawson bzw. keinen Farbstoff enthält:
Die so erhaltenen Farbtöne werden nach 21,5 Stunden (Tabelle 3) ausgewertet.

Die erfindungsgemäße Zusammensetzung führt zu einem niedrigen Bunttonwinkel h, d.h. zu einer Rotbraun-Färbung als die Lawson-haltige Zusammensetzung, die einen gelblichbraunen Farbton hervorruft:

Tabelle 3

| Formulierung | 5 % DHA | 5 % DHA + Lawson | 5 % DHA + Eosin G |
|---|---|---|---|
| h | 65,2241 | 78,3972 | 59,2559 |

Beispiele 2-7: Getönte Selbstbräunungscremes

| | | | % |
|---|---|---|---|
| A | Emulgator E 2155<br>Paraffinöl flüssig (Art.-Nr. 7162)<br>Paraffin schüttfähig (Art.-Nr. 7158)<br>Miglyol 812<br>Isopropylmyristat | (2)<br>(1)<br>(1)<br>(3)<br>(4) | 8,000<br>12,000<br>2,000<br>3,000<br>2,000 |
| B | Propandiol-1,2 (Art.-Nr. 7478)<br>Karion F flüssig (Art.-Nr. 2993)<br>Farbstoffmischung (gemäß Tabelle 4)<br>Konservierungsmittel<br>Wasser, demineralisiert | (1)<br>(1) | 4,000<br>2,000<br>5,000<br>q.s.<br>ad 100,000 |
| C | Dihydroxyaceton (Art.-Nr. 10150)<br>Wasser, demineralisiert | (1) | 5,000<br>11,800 |

Konservierungsmittel:

0,05 % Propyl-4-hydroxybenzoat (Merck-Art.-Nr. 7427)
0,15 % Methyl-4-hydroxybenzoat (Merck-Art.-Nr. 6757)

Bezugsquellen:

1. E. Merck, Darmstadt
2. Th. Goldschmidt, Essen
3. Hüls Troisdorf AG, Witten
4. Henkel, Düsseldorf

Herstellung:

Phase A auf 75 °C, Phase B auf 80 °C erhitzen. Phase B langsam in Phase A einrühren. Homogenisieren. Rühren, bei 40 °C Phase C zugeben.

Tabelle 4

| Farbstoffmischungen | | | |
|---|---|---|---|
| Beispiel | Eosin G | Tatrazin | Solagenbrillantschwarz |
| 2 | 3 % | 6,2 % (11,4 %) | 1,24 % (2,28 %) |
| 3 | 5 % | 6,2 % (11,4 %) | 1,24 % (2,28 %) |
| 4 | 7 % | 6,2 % (11,4 %) | 1,24 % (2,28 %) |
| Beispiel | Erythrosin I | Tatrazin | Solagenbrillantschwarz |
| 5 | 2 % | 11,4 % | 2 % |
| 6 | 4 % | 11,4 % | 2 % |
| 7 | 6 % | 11,4 % | 2 % |

Bemerkung

Die Lösungen sind 1%ig und werden in die wäßrige Phase gegeben.
Eosin G (C.I. 45380)
Solagenbrillantschwarz (C.I. 28440)

Tatrazin (C.I. 19140)
Erythrosin I (C.I. 45439)
Alle Farbstoffe sind in der Blauen Liste aufgeführt.

## Patentansprüche

1. Hautfärbende Zubereitung, enthaltend eine Hydroxycarbonyl-Verbindung mit selbstbräunenden Eigenschaften, in einem kosmetisch verträglichen Träger, dadurch gekennzeichnet, daß sie mindestens einen hauthaftenden Farbstoff enthält.

2. Hautfärbende Zubereitung nach Anspruch 1, dadurch gekennzeichnet, daß die Verbindung mit selbstbräunenden Eigenschaften Dihydroxyaceton oder Methylglyoxal ist.

3. Hautfärbende Zubereitung nach Anspruch 1 oder 2, dadurch gekennzeichnet, daß der hauthaftende Farbstoff ein roter Farbstoff, vorzugsweise ein Eosin-Derivat, ist.

4. Hautfärbende Zubereitung nach einem der Ansprüche 1 bis 3, dadurch gekennzeichnet, daß sie 0,1 bis 10, vorzugsweise 1 bis 6 Gew.-% einer hautfärbenden Verbindung, und 0,01 bis 0,1, vorzugsweise etwa 0,05 Gew.-% mindestens eines hauthaftenden Farbstoffes jeweils bezogen auf die gesamte Zubereitung enthält.

5. Hautfärbende Zubereitung nach einem der Ansprüche 1 bis 4, dadurch gekennzeichnet, daß das Gewichtsverhältnis zwischen dem hauthaftenden Farbstoff und der hautfärbenden Verbindung größer als 0,01 ist.

6. Hautfärbende Zubereitung nach einem der Ansprüche 1 bis 5, dadurch gekennzeichnet, daß der kosmetisch verträgliche Träger ausgewählt ist aus der Gruppe bestehend aus:
   Wasser;
   einem Gemisch aus Wasser und einem oder mehreren organischen Komponenten;
   einem Gemisch aus Fetten und einem oder mehreren organischen Lösungsmitteln.

7. Hautfärbende Zubereitung nach einem der Ansprüche 1 bis 6, dadurch gekennzeichnet, daß der pH-Wert zwischen 2 und 10, vorzugsweise zwischen 3 und 6, liegt:

8. Hautfärbende Zubereitung nach einem der Ansprüche 1 bis 7, dadurch gekennzeichnet, daß sie mindestens einen UV-Filter zum Schutz vor UV-A und/oder UV-B Strahlen enthält.

9. Hautfärbende Zubereitung nach einem der Ansprüche 1 bis 8, dadurch gekennzeichnet, daß sie oberflächenaktive Mittel, feuchtigkeitsspendende Mittel, Linderungsmittel, Trübungsmittel, Dihydroxyindol-Derivate, Vitamine, Parfüme, Gelbildner, Verdickungsmittel, Pigmente, vorzugsweise Effekt-Pigmente, insbesondere Perlglanzpigmente oder transparente Pigmente, den Farbton des hauthaftenden Farbstoffes abdeckende Farbstoffe oder Konservierungsmittel enthält.

10. Verfahren zur Färbung der Haut mit einem der natürlichen Bräunung ähnlichem Farbton, dadurch gekennzeichnet, daß man eine Zubereitung gemäß einem der Ansprüche 1 bis 9 auf die Haut aufbringt.

Europäisches Patentamt

# EUROPÄISCHER RECHERCHENBERICHT

Nummer der Anmeldung
EP 94 10 7899

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| A,D | EP-A-0 500 446 (L'OREAL)<br>* das ganze Dokument *<br>--- | 1-10 | A61K7/42 |
| A,D | US-A-3 920 808 (FUSARO)<br>* das ganze Dokument *<br>--- | 1-10 | |
| A | EP-A-0 431 210 (BRISTOL MYERS SQUIBB COMPANY)<br>* das ganze Dokument *<br>--- | 1-10 | |
| A | FR-A-603 263 (PERL)<br>* das ganze Dokument *<br>--- | 1-10 | |
| A | US-A-3 749 768 (KALOPISSIS ET AL.)<br>* das ganze Dokument *<br>----- | 1-10 | |

RECHERCHIERTE SACHGEBIETE (Int.Cl.5)

A61K

Der vorliegende Recherchenbericht wurde für alle Patentansprüche erstellt

| Recherchenort | Abschlußdatum der Recherche | Prüfer |
|---|---|---|
| DEN HAAG | 21. Juni 1994 | Fischer, J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
................................................................
& : Mitglied der gleichen Patentfamilie, übereinstimmendes Dokument

EPO FORM 1503 03.82 (P04C03)